# EUROPÄISCHE PATENTSCHRIFT

(11) **EP 3 221 288 B1**
(45) Veröffentlichungstag und Bekanntmachung des Hinweises auf die Patenterteilung: **26.12.2018**
(21) Anmeldenummer: 14801998.7
(22) Anmeldetag: 18.11.2014
(51) Int. Cl.: C07C 51/23, C07C 51/41, C07C 51/46

(54) **VERFAHREN ZUM GEWINNEN EINES FORMIATS AUS EINEM REAKTIONSGEMISCH**
PROCESS FOR OBTAINING A FORMATE FROM A REACTION MIXTURE
PROCÉDÉ D'OBTENTION D'UN FORMIATE À PARTIR D'UN MÉLANGE RÉACTIONNEL

(43) Veröffentlichungstag der Anmeldung: 27.09.2017
(73) Patentinhaber: Jbach GmbH, 96120 Bischberg (DE)
(72) Erfinder: JBACH, Hermann Wolf, 96120 Bischberg (DE); PLATTEN, Carmen, 97478 Knetzgau (DE); SCHOLZ, Gunthard, 96163 Gundelsheim (DE); BÖSMANN, Andreas, 91093 Heßdorf (DE)
(74) Vertreter: Dr. Gassner & Partner mbB
(86) Internationale Anmeldenummer: PCT/EP2014/074930
(87) Internationale Veröffentlichungsnummer: WO 2016/078698

(56) Entgegenhaltungen:
- WO-A1-2012/034839
- WO-A2-2009/130387

## Beschreibung

Die Erfindung betrifft ein Verfahren zum Gewinnen eines Formiats aus einem Reaktionsgemisch, in welchem ein als Katalysator dienendes Polyoxometallat-Ion bei einer Temperatur unterhalb von 120°C mit einem organischen Stoff unter Entstehung von Ameisensäure in einer wässrigen Lösung in Kontakt steht.

Ein solches Reaktionsgemisch ist aus der WO 2012/034839 A1 bekannt. Die WO 2012/034839 A1 betrifft ein Verfahren zur katalytischen Erzeugung von Ameisensäure, wobei ein als Katalysator dienendes Polyoxometallat-Ion der allgemeinen Formel [PMoₓV_{y}O₄₀]⁵⁻ bei einer Temperatur unterhalb von 120°C mit einem alpha-Hydroxyaldehyd, einer alpha-Hydroxycarbonsäure, einem Kohlenhydrat oder einem Glycosid in einer flüssigen Lösung in Kontakt gebracht wird. Dabei ist 6 < x < 11 und 1 < y < 6 und x + y = 12, wobei x und y jeweils eine ganze Zahl ist. Eine solche Lösung kann als Reaktionsgemisch auch beim erfindungsgemäßen Verfahren eingesetzt werden. Die bei dem Verfahren gemäß der WO 2012/034839 A1 erzeugte Ameisensäure kann durch Destillation, Reaktivdestillation, Extraktion, insbesondere zusammen mit dem Katalysator, insbesondere durch Umsetzen mit einer Base, insbesondere einem Amin, oder durch Strippen aus der Lösung entfernt werden. Zur Extraktion kann ein Ether oder ein Amid zugesetzt werden. Ein Verfahren, mit dem die entstehende Ameisensäure kontinuierlich während der Durchführung des Verfahrens aus dem Reaktionsgemisch entfernt werden kann, wird jedoch nicht angegeben.

Aus der US 2011/0098490 A1 und der WO 2009/130387 A2 ist ein Verfahren zum Absondern und Rückgewinnen eines Formiats aus einem wässrigen Flüssigkeitsgemisch, welches Ameisensäure, Lävulinsäure und optional Furfural enthält, bekannt. Dabei wird das Gemisch einer Flüssig-Flüssig-Extraktion unterworfen, wobei daraus eine organische Phase mit dem Extraktionsmittel, Ameisensäure, Lävulinsäure und optional Furfural und eine Wasser und mindestens eine anorganische Säure umfassende wässrige Phase resultiert. Die wässrige Phase wird von der organischen Phase getrennt. Ameisensäure wird von der organischen Phase durch Destillation abgetrennt und in Form eines Formiats aus der organischen Phase durch nachfolgende Neutralisation gewonnen.
Aus der EP 0 038 317 B1 ist ein Verfahren zur Gewinnung von im Wesentlichen Furfurol, Ameisensäure, Essigsäure und anderen organischen Verbindungen aus sauren Hydrolysaten von Pflanzen bekannt. Dabei wird einem Reaktor Hydrolysat zugeführt und dort auf eine Temperatur von über 200°C erhitzt. Furfurol, Ameisensäure und Essigsäure wird dann als Destillat gewonnen und dann ggf. mit einem Extraktionsmittel extrahiert. Bei einer solchen Temperatur kommt es jedoch bereits zu einem Zerfall von Ameisensäure z. B. zu Kohlenmonoxid und Wasser.
Keines der genannten Dokumente offenbart ein Verfahren, welches ein kontinuierliches Gewinnen eines Formiats aus einem Reaktionsgemisch, in welchem Ameisensäure gebildet wird, erlaubt. Unter "kontinuierlichem Gewinnen eines Formiats" wird dabei eine Formiatgewinnung verstanden, die zumindest während eines überwiegenden Teils derjenigen Zeit erfolgt, in der auch Ameisensäure im Reaktionsgemisch entsteht. Aufgabe der vorliegenden Erfindung ist es, ein Verfahren anzugeben, welches ein kontinuierliches Gewinnen eines Formiats aus einem Reaktionsgemisch, in dem gleichzeitig Ameisensäure gebildet wird, ermöglicht.
Die Aufgabe wird durch die Merkmale des Patentanspruchs 1 gelöst. Zweckmäßige Ausgestaltungen ergeben sich aus den Merkmalen der Patentansprüche 2 bis 10. Erfindungsgemäß ist ein Verfahren zum Gewinnen eines Formiats aus einem Reaktionsgemisch vorgesehen. In dem Reaktionsgemisch steht ein als Katalysator dienendes Polyoxometallat-Ion der allgemeinen Formel [PMoₓV_{y}O₄₀]ⁿ⁻ bei einer Temperatur unterhalb von 120°C mit einem organischen Stoff unter Entstehung von Ameisensäure in einer wässrigen Lösung in Kontakt. Dabei ist 6 ≤ x ≤ 11, 1 ≤ y ≤ 6, x + y = 12 und 3 < n < 10, wobei n, x und y jeweils eine ganze Zahl ist. Bei einer Ausgestaltung des Verfahrens ist n = 3 + y. Die Ladung n kann jedoch in Abhängigkeit von den Bedingungen in dem Reaktionsgemisch, wie z. B. dem pH-Wert, auch andere ganzzahlige Werte von 4 bis 9 annehmen. Unter einem Polyoxometallat-Ion wird in diesem Sinne auch eine Mehrzahl identischer Polyoxometallat-Ionen verstanden. Das Verfahren umfasst die folgenden Schritte:
a) Absondern eines Gemischs aus Ameisensäure und Wasser aus dem Reaktionsgemisch durch Umkehrosmose und/oder als Dampf, wobei der Dampf anschließend kondensiert wird und
b) Umsetzen der nicht vom Wasser abgesonderten Ameisensäure mit einem Hydroxid in wässriger Lösung unter Entstehung einer Lösung eines Formiats,
wobei es sich bei dem organischen Stoff um ein alpha-Hydroxyaldehyd, eine alpha-Hydroxycarbonsäure, ein Kohlenhydrat oder ein Glycosid handelt. Das Kondensieren des Dampfs kann mit Hilfe eines Kühlers für Destillationszwecke, beispielsweise eines Liebigkühlers, erfolgen. Die Ameisensäure bei Schritt b) ist die Ameisensäure in dem gemäß Schritt a) abgesonderten Gemisch aus Ameisensäure und Wasser. Die Ameisensäure braucht zum Umsetzen mit dem Hydroxid nicht von dem Gemisch aus Ameisensäure und Wasser abgesondert zu werden. Die bei Schritt b) entstandene Lösung kann in einem Schritt c) durch Absondern von Wasser durch Umkehrosmose und/oder teilweises oder vollständiges Verdampfen des Wassers konzentriert werden. Das Verdampfen bei Schritt c) kann bis zum Erreichen der Trockenheit des Formiats erfolgen. Dem Reaktionsgemisch kann ein Tensid oder ein sonstiges Additiv, insbesondere eine Sulfonsäure, insbesondere Methansulfonsäure, Trifluormethansulfonsäure, Camphersulfonsäure, Toluolsulfonsäure, insbesondere para-Toluolsulfonsäure, Chlorsulfonsäure, Xylolsulfonsäure, Benzolsulfonsäure oder ein Derivat einer der genannten Säuren, insbesondere Chlorbenzolsulfonsäure, insbesondere para-Chlorbenzolsulfonsäure, oder ein Salz einer der genannten Säuren oder des Derivats oder ein sonstiger Stoff, der in einer wässrigen Lösung eine der genannten Säuren oder das Derivat bildet, zugesetzt sein. Dadurch kann der organische Stoff, insbesondere Altpapier, Holz oder Bakterien, besser umgesetzt werden. Insgesamt kann dadurch eine bessere Ausbeute erzielt werden.

Der Erfinder der vorliegenden Erfindung hat erkannt, dass sich Formiate, welche in großen Mengen, beispielsweise als Enteisungsmittel, kommerziell gehandelt werden, sehr viel einfacher direkt herstellen lassen als über den Umweg der Isolierung von Ameisensäure. Er hat weiterhin erkannt, dass es zur Gewinnung von Formiaten in einem ersten Reaktionsschritt nicht erforderlich ist, Ameisensäure von dem damit ein azeotropes Gemisch bildenden Wasser abzusondern. Der Erfinder hat auch erkannt, dass es ohne weiteres möglich ist, ein Ameisensäuredampf-Wasserdampf-Gemisch aus einem Reaktionsgemisch bei einer Temperatur unterhalb von 120°C abzusondern, wobei das Reaktionsgemisch einen organischen Stoff und ein als Katalysator dienendes Polyoxometallat-Ion umfasst, welche beide beim Absondern im Reaktionsgemisch verbleiben. Das Reaktionsgemisch kann dabei eine flüssige Lösung sein, wie sie beim Verfahren zur katalytischen Erzeugung von Ameisensäure gemäß der WO 2012/034839 A1 eingesetzt wird. Der große Vorteil des Verfahrens besteht dabei darin, dass die eigentliche Reaktion, bei der Ameisensäure in dem Reaktionsgemisch entsteht, zum Absondern des Dampfgemischs nicht unterbrochen zu werden braucht und kontinuierlich fortgesetzt werden kann. Durch ein kontinuierliches Entfernen von Ameisensäure aus dem Reaktionsgleichgewicht wird auch die weitere Entstehung von Ameisensäure unterstützt.

Bei einer Ausgestaltung des erfindungsgemäßen Verfahrens wird dem Reaktionsgemisch zum Ausgleich des bei Schritt a) abgesonderten Wassers weiteres Wasser und/oder das bei Schritt c) abgesonderte Wasser zugesetzt. Dadurch kann ein zur Bildung von Ameisensäure erforderliches oder günstiges Konzentrationsverhältnis in dem Reaktionsgemisch während der Durchführung des Verfahrens aufrechterhalten werden.

Bei einer weiteren Ausgestaltung des erfindungsgemäßen Verfahrens wird dem Reaktionsgemisch zum Ausgleich von umgesetztem organischem Stoff weiterer organischer Stoff zugesetzt. Der weitere organische Stoff kann dabei mit dem ursprünglich im Reaktionsgemisch enthaltenen organischen Stoff identisch sein. Das kontinuierliche Gewinnen des Formiats kann dadurch auf einen längeren Zeitraum ausgedehnt werden.
Das Inkontaktstehen des organischen Stoffs mit dem Katalysator und/oder das Absondern als Dampf gemäß Schritt a) kann bei einer Temperatur von 15 bis 119,5°C, insbesondere 40 bis 95°C, insbesondere 50 bis 85°C, insbesondere 55 bis 85°C, insbesondere 60 bis 83°C, erfolgen. Bei dieser Temperatur ist die Reaktionstemperatur im Reaktionsgemisch ausreichend hoch, um eine schnelle Bildung von Ameisensäure zu ermöglichen. Günstig ist eine Temperatur im Bereich von 60 bis 83°C, da hier der Unterschied im Dampfdruck zwischen Ameisensäure und Wasser am größten ist, wobei Ameisensäure den höheren Dampfdruck aufweist. Dadurch ist in einem bei dieser Temperatur abgesonderten Ameisensäuredampf-Wasserdampf-Gemisch der Anteil an Ameisensäuredampf verhältnismäßig hoch. Um bei einer Temperatur unter 100°C ein effektives Verdampfen zu ermöglichen, ist eine Reduktion des auf dem Reaktionsgemisch lastenden Drucks erforderlich. Bei einer Ausgestaltung des erfindungsgemäßen Verfahrens erfolgt das Absondern als Dampf gemäß Schritt a) daher unter gegenüber dem Atmosphärendruck reduziertem Druck.

Bei dem organischen Stoff handelt es sich um ein alpha-Hydroxyaldehyd, eine alpha-Hydroxycarbonsäure, ein Kohlenhydrat oder ein Glycosid, wie es aus der WO 2012/034839 A1 bekannt ist. Alpha-Hydroxyaldehyde, Kohlenhydrate und Glycoside kommen in einer großen Zahl der nachwachsenden Rohstoffe, wie beispielsweise Stärke, Zellulose oder Hemizellulose vor. Stärke, Zellulose und Hemizellulose fallen in großen Mengen als Produkt aus Ackerpflanzen oder beim industriellen Holzaufschluss, beispielsweise zur Papierherstellung an.
Bei dem alpha-Hydroxyaldehyd, der alpha-Hydroxycarbonsäure, dem Kohlenhydrat oder dem Glycosid kann es sich um ein Monosaccharid, insbesondere einer Aldose, Disaccharid, Oligosaccharid oder Polysaccharid, Stärke, Zellulose, Hemizellulose, Glucose, Saccharose, Xylose, Zellobiose, Xylan, ein Hetereooligosaccharid, ein Heteropolysaccharid, Glycolsäure oder Milchsäure oder einen das alpha-Hydroxyaldehyd, die alpha-Hydroxycarbonsäure, das Kohlenhydrat oder das Glycosid enthaltenden Reststoffen oder, insbesondere nachwachsenden, insbesondere unbehandelten, Rohstoff handeln. Unbehandelt bedeutet dabei, dass dieser zuvor nicht chemisch aufgeschlossen worden ist. Bei dem Reststoff oder dem nachwachsenden Rohstoff kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage handeln. Das alpha-Hydroxyaldehyd, die alpha-Hydroxycarbonsäure, das Kohlenhydrat oder das Glycosid kann auch ein Gemisch aus mindestens zwei der genannten Substanzen umfassen oder aus mindestens einer der genannten Substanzen oder dem Gemisch entstanden sein.

Viele der genannten Rohstoffe fallen als Nebenprodukte, beispielsweise bei der Papierherstellung oder Holzverarbeitung an. Sie stehen damit als günstiger Ausgangsstoff für das erfindungsgemäße Verfahren zur Verfügung. Das erfindungsgemäße Verfahren kann dadurch sehr kostengünstig durchgeführt werden.

Einen besonders geringen apparativen Aufwand erfordert das erfindungsgemäße Verfahren, wenn das Kondensieren des Dampfes gemäß Schritt a) durch Einleiten des Dampfes in Wasser, eine wässrige Lösung oder die wässrige Lösung gemäß Schritt b), d. h. eine wässrige Hydroxidlösung, erfolgt. Im Falle einer wässrigen Hydroxidlösung entsteht dabei sofort das gewünschte Formiat. Bei dem Hydroxid kann es sich um Ammoniumhydroxid, Aluminiumhydroxid, Kupferhydroxid, Nickelhydroxid, ein Erdalkalimetallhydroxid, ein Alkalimetallhydroxid, Calciumhydroxid, Magnesiumhydroxid, Natriumhydroxid, Kaliumhydroxid oder Cäsiumhydroxid handeln. Ammoniumhydroxid hat den besonderen Vorteil, dass nicht mit Ameisensäure umgesetztes Ammoniumhydroxid bei oder nach Schritt c) durch Verdampfen vom entstandenen Ammoniumformiat abgesondert werden kann. Ein Absondern von nicht umgesetztem Hydroxid ist nicht erforderlich, wenn das Hydroxid bei Schritt b) in einem solchen stöchiometrischen Verhältnis zur Ameisensäure eingesetzt wird, dass es vollständig oder zumindest nahezu vollständig umgesetzt wird.

Eine vollständige Umsetzung des Hydroxids kann auch dadurch erreicht werden, dass es im Unterschuss im Verhältnis zur Ameisensäure eingesetzt wird. Dabei verbleibende nicht umgesetzte Ameisensäure stört je nach vorgesehener Anwendung nicht oder kann bei Schritt c) zusammen mit dem Wasser abgesondert werden. Bei einer nahezu vollständigen Umsetzung des Hydroxids verbleibt zwar ein Rest des Hydroxids im gebildeten Formiat, je nach vorgesehener Anwendung stört jedoch auch dies nicht.

Nachfolgend wird die Erfindung anhand einer Zeichnung und eines Ausführungsbeispiels näher erläutert:
Es zeigen
- Fig. 1: eine schematische Darstellung eines einfach ausgestalteten erfindungsgemäßen Verfahrens und
- Fig. 2: eine schematische Darstellung einer weiteren Ausgestaltung des erfindungsgemäßen Verfahrens.

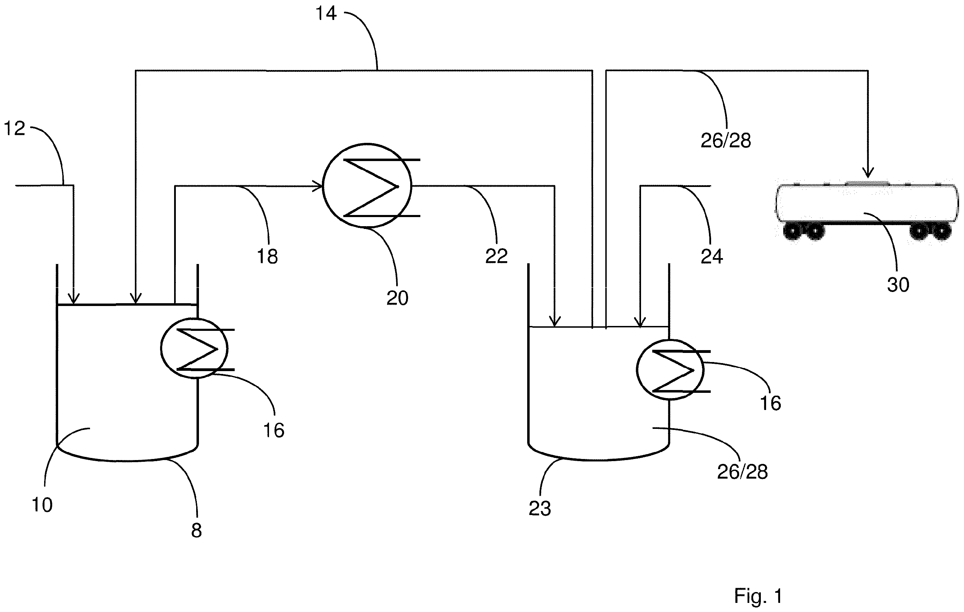

Die schematische Darstellung gemäß Fig. 1 zeigt ein erstes Gefäß 8, in welchem sich ein Reaktionsgemisch 10 befindet. Das Reaktionsgemisch 10 umfasst Wasser, Polyoxometallat-Ionen als Katalysator, einen organischen Stoff und aus dem organischen Stoff mittels des Katalysators gebildete Ameisensäure. Das Reaktionsgemisch wird mittels eines Wärmetauschers16 in einem Temperaturbereich von etwa 78°C bis 82°C gehalten. Je nach eingesetztem Substrat, eingesetztem Katalysator, eingesetzter Katalysatormenge und Oxidationsgrad des eingesetzten Katalysators kann es dazu erforderlich sein, dem Reaktionsgemisch 10 Wärme zuzuführen oder zu entziehen. Der Druck in dem Reaktionsgemisch ist so weit unterhalb des Atmosphärendrucks abgesenkt, dass ein Ameisensäuredampf-Wasserdampf-Gemisch 18 in diesem Temperaturbereich effektiv verdampft. Das Ameisensäuredampf-Wasserdampf-Gemisch 18 wird mittels des Kühlers 20 zu dem Ameisensäure-Wasser-Gemisch 22 kondensiert, welches in ein zweites Gefäß 23 eingebracht wird. Zum Ausgleich des bei der Bildung der Ameisensäure umgesetzten organischen Stoffs wird weiterer organischer Stoff 12 zugeführt.

Zu dem Ameisensäure-Wasser-Gemisch 22 im zweiten Gefäß 23 wird ein Hydroxid 24, beispielsweise in Form einer Kaliumhydroxidlösung oder eines festen Kaliumhydroxids, zugesetzt. In dem zweiten Gefäß 23 erfolgt dadurch eine exotherme Neutralisation der Ameisensäure, wobei ein Formiat gebildet wird. Sofern und soweit es wegen der exothermen Reaktion noch erforderlich ist wird das resultierende Formiat-Wasser-Gemisch 26 mittels des Wärmetauschers 16 erhitzt, so dass Wasserdampf 14 verdampft und nach Kondensation in einem hier nicht dargestellten Kühler wieder dem Reaktionsgemisch 10 zugeführt werden kann. In dem zweiten Gefäß 23 verbleibt Formiat bzw. eine konzentrierte Formiat-Lösung 28, welche(s) zum Abtransport beispielsweise in einen Transport- bzw. Tankwagen 30 gefüllt werden kann.

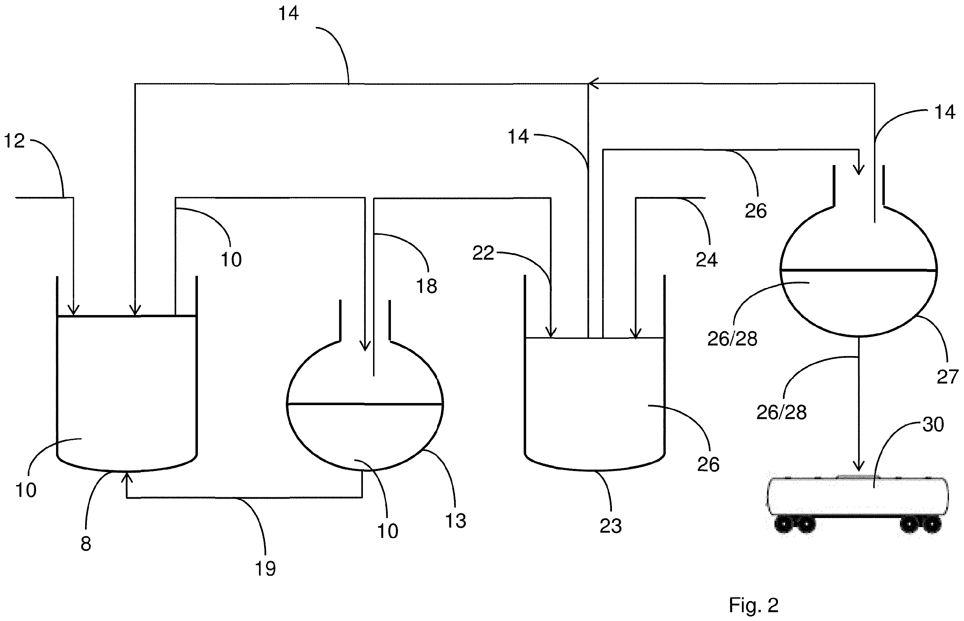

Bei der weiteren Ausgestaltung des erfindungsgemäßen Verfahrens gemäß Fig. 2 wird aus dem das Reaktionsgemisch 10 enthaltenden ersten Gefäß 8, welches mittels eines hier nicht dargestellten Wärmetauschers in einem Temperaturbereich von etwa 92°C bis 97°C gehalten wird, ein Teil des Reaktionsgemischs 10 entnommen und in ein erstes Destillationsgefäß 13 überführt. Dort wird das Reaktionsgemisch 10 unter gegenüber dem Atmosphärendruck reduziertem Druck in einem Temperaturbereich von etwa 78°C bis 82°C mittels eines hier ebenfalls nicht dargestellten Wärmetauschers gehalten, so dass ein Ameisensäuredampf-Wasserdampf-Gemisch 18 entsteht. Das Ameisensäuredampf-Wasserdampf-Gemisch 18 entweicht dem ersten Destillationsgefäß 13 über eine Leitung und wird in einem hier nicht dargestellten Kühler zu einem Ameisensäure-Wasser-Gemisch 22 kondensiert und dem zweiten Gefäß 23 zugeführt. Der verbleibende Wasser, Polyoxometallat-Ionen und organischen Stoff sowie einen Rest von Ameisensäure enthaltende Rückstand 19 wird wieder dem Reaktionsgemisch 10 im ersten Gefäß zugeführt. Zum Ausgleich für bei der Reaktion umgesetzten organischen Stoff wird dem Reaktionsgemisch 10 weiterer organischer Stoff 12 zugeführt.

Dem Ameisensäure-Wasser-Gemisch 22 im zweiten Gefäß 23 wird ein Hydroxid 24 zugefügt, so dass sich bei der dadurch erfolgenden Neutralisation ein Formiat bildet. Da diese Neutralisation exotherm ist, erwärmt sich das Formiat-Wasser-Gemisch 26 dabei. Entstehender Wasserdampf 14 kann nach Kondensation mittels eines hier nicht dargestellten Kühlers wieder dem Reaktionsgemisch 10 zugeführt werden. Das Formiat-Wasser-Gemisch 26 aus dem zweiten Gefäß 23 wird einem zweiten Destillationsgefäß 27 zugeführt. Sofern und soweit es erforderlich ist wird das Formiat-Wasser-Gemisch 26 mittels einer hier nicht dargestellten Heizung erhitzt, so dass in dem Formiat-Wasser-Gemisch 26 enthaltenes Wasser 14, ggf. unter reduziertem Druck, als Wasserdampf 14 entweicht. Nach Kondensation in einem hier nicht dargestellten Kühler wird das Wasser 14 dem Reaktionsgemisch 10 zugeführt. Das Formiat-Wasser-Gemisch 26 kann in dem zweiten Destillationsgefäß 27 so lange eingedampft werden, bis das Formiat in kristalliner Form vorliegt. Das verbleibende Formiat bzw. die konzentrierte Formiat-Lösung 28 wird zum Abtransport aus dem zweiten Destillationsgefäß 27 in einen Transport- bzw. Tankwagen 30 überführt.

Das Verfahren gemäß Fig. 2 ist gegenüber dem Verfahren gemäß Fig. 1 zwar apparativ aufwändiger, hat jedoch den Vorteil, dass die Temperatur beim Verdampfen des Ameisensäuredampf-Wasserdampf-Gemischs 18 unabhängig von der Reaktionstemperatur im ersten Gefäß 8 gewählt werden kann. So kann die Temperatur und damit die Reaktionsgeschwindigkeit im ersten Gefäß 8 höher gewählt werden, als es für ein Verdampfen eines Ameisensäuredampf-Wasserdampf-Gemischs 18 mit einem möglichst hohen Ameisensäuredampfanteil günstig ist.

### Bezugszeichenliste

- 8: erstes Gefäß
- 10: Reaktionsgemisch
- 12: weiterer organischer Stoff
- 13: erstes Destillationsgefäß
- 14: Wasser/Wasserdampf
- 16: Wärmetauscher
- 18: Ameisensäuredampf-Wasserdampf-Gemisch
- 19: Rückstand
- 20: Kühler
- 22: Ameisensäure-Wasser-Gemisch
- 23: zweites Gefäß
- 24: Hydroxid
- 26: Formiat-Wasser-Gemisch
- 27: zweites Destillationsgefäß
- 28: konzentrierte Formiat-Lösung
- 30: Tankwagen

## Patentansprüche

1. Verfahren zum Gewinnen eines Formiats aus einem Reaktionsgemisch (10), in welchem ein als Katalysator dienendes Polyoxometallat-Ion der allgemeinen Formel [PMoₓV_{y}O₄₀]ⁿ⁻ bei einer Temperatur unterhalb von 120°C mit einem organischen Stoff unter Entstehung von Ameisensäure in einer wässrigen Lösung in Kontakt steht, wobei 6 ≤ x ≤ 11, 1 ≤ y ≤ 6, x + y = 12 und 3 < n < 10, wobei n, x und y jeweils eine ganze Zahl ist, mit folgenden Schritten:
a) Absondern eines Gemischs aus Ameisensäure und Wasser aus dem Reaktionsgemisch durch Umkehrosmose und/oder als Dampf (18), wobei der Dampf (18) anschließend kondensiert wird und
b) Umsetzen der nicht vom Wasser abgesonderten Ameisensäure mit einem Hydroxid (24) in wässriger Lösung unter Entstehung einer Lösung eines Formiats,
wobei es sich bei dem organischen Stoff um ein alpha-Hydroxyaldehyd, eine alpha-Hydroxycarbonsäure, ein Kohlenhydrat oder ein Glycosid handelt.

2. Verfahren nach Anspruch 1, wobei die bei Schritt b) entstandenen Lösung in einem Schritt c) durch Absondern von Wasser (14) durch Umkehrosmose und/oder teilweises oder vollständiges Verdampfen des Wassers (14) konzentriert wird.

3. Verfahren nach einem der vorhergehenden Ansprüche, wobei dem Reaktionsgemisch (10) zum Ausgleich des bei Schritt a) abgesonderten Wassers weiteres Wasser und/oder das bei Schritt c) abgesonderte Wasser (14) zugesetzt wird.

4. Verfahren nach einem der vorhergehenden Ansprüche, wobei dem Reaktionsgemisch (10) zum Ausgleich von umgesetztem organischem Stoff weiterer organischer Stoff (12) zugesetzt wird.

5. Verfahren nach einem der vorhergehenden Ansprüche, wobei das Inkontaktstehen und/oder das Absondern als Dampf (18) gemäß Schritt a) bei einer Temperatur von 15 bis 119,5°C, insbesondere 40 bis 95°C, insbesondere 50 bis 85°C, insbesondere 55 bis 85°C, insbesondere 60 bis 83°C, erfolgt.

6. Verfahren nach einem der vorhergehenden Ansprüche, wobei das Absondern als Dampf (18) gemäß Schritt a) unter gegenüber dem Atmosphärendruck reduziertem Druck erfolgt.

7. Verfahren nach einem der vorhergehenden Ansprüche, wobei das Kondensieren des Dampfes (18) gemäß Schritt a) durch Einleiten des Dampfes (18) in Wasser, eine wässrige Lösung oder die wässrige Lösung gemäß Schritt b) erfolgt.

8. Verfahren nach einem der vorhergehenden Ansprüche, wobei das Hydroxid (24) Ammoniumhydroxid, Aluminiumhydroxid, Kupferhydroxid, Nickelhydroxid, ein Erdalkalimetallhydroxid, ein Alkalimetallhydroxid, Calciumhydroxid, Magnesiumhydroxid, Natriumhydroxid, Kaliumhydroxid oder Cäsiumhydroxid ist.

9. Verfahren nach einem der vorhergehenden Ansprüche, wobei das Hydroxid (24) bei Schritt b) in einem solchen stöchiometrischen Verhältnis zur Ameisensäure eingesetzt wird, dass es vollständig oder nahezu vollständig umgesetzt wird.

10. Verfahren nach einem der vorhergehenden Ansprüche, wobei n = 3 + y.

## Claims

1. A method for obtaining a formate from a reaction mixture (10), in which a polyoxometallate ion, which acts as a catalyst, of the general formula [PMoₓV_{y}O₄₀]ⁿ⁻ is in contact with an organic material at a temperature below 120 °C to produce formic acid in an aqueous solution, wherein 6 ≤ x ≤ 11, 1 ≤ y ≤ 6, x + y = 12 and 3 < n < 10, wherein n, x and y are each a whole number, which method comprises the following steps:
a) separating a mixture of formic acid and water from the reaction mixture by reverse osmosis and/or as vapor (18), the vapor 18 subsequently being condensed and
b) reacting the formic acid not separated from the water with a hydroxide (24) in aqueous solution to produce a solution of a formate,
wherein the organic material is an alpha-hydroxyaldehyde, an alpha-hydroxycarboxylic acid, a carbohydrate, or a glycoside.

2. The method according to claim 1, wherein the solution produced in step b) is concentrated in a step c) by separation of water (14) by reverse osmosis and/or partial or complete evaporation of the water (14).

3. The method according to any one of the preceding claims, wherein further water and/or the water (14) separated in step c) is added to the reaction mixture (10) to compensate for the water separated in step a).

4. The method according to any one of the preceding claims, wherein further organic material (12) is added to the reaction mixture (10) to compensate for the reacted organic material.

5. The method according to any one of the preceding claims, wherein the maintenance of contact and/or separation as vapor (18) according to step a) occurs at a temperature of 15 to 119.5°C, in particular 40 to 95°C, in particular 50 to 85°C, in particular 55 to 85°C, in particular 60 to 83°C.

6. The method according to any one of the preceding claims, wherein the separation as vapor (18) according to step a) takes place at a pressure that is reduced with respect to atmospheric pressure.

7. The method according to any one of the preceding claims, wherein the condensation of the vapor (18) according to step a) occurs by introducing the vapor (18) into water, an aqueous solution, or the aqueous solution according to step b).

8. The method according to any one of the preceding claims, wherein the hydroxide (24) is ammonium hydroxide, aluminum hydroxide, copper hydroxide, nickel hydroxide, an alkaline earth metal hydroxide, an alkali metal hydroxide, calcium hydroxide, magnesium hydroxide, sodium hydroxide, potassium hydroxide, or cesium hydroxide.

9. The method according to any one of the preceding claims, wherein the hydroxide (24) in step b) is used in such a stoichiometric ratio to the formic acid that it is reacted completely or almost completely.

10. The method according to any one of the preceding claims, wherein n = 3 + y.

## Revendications

1. Procédé d'obtention d'un formiate à partir d'un mélange réactionnel (10), dans lequel un ion polyoxométallate servant de catalyseur de la formule générale [PMoₓV_{y}O₄₀]ⁿ⁻ est en contact avec une substance organique à une température en dessous de 120°C avec la formation d'acide formique dans une solution aqueuse, dans lequel 6 ≤ x ≤ 11, 1 ≤ y ≤ 6, x + y = 12 et 3 < n < 10, dans lequel n, x et y sont chacun un nombre entier, avec les étapes suivantes :
a) séparation d'un mélange d'acide formique et d'eau du mélange réactionnel par osmose inverse et/ou en tant que vapeur (18), dans lequel la vapeur (18) est ensuite condensée, et
b) transformation de l'acide formique non séparé de l'eau avec un hydroxyde (24) en solution aqueuse avec la formation d'une solution d'un formiate,
dans lequel il s'agit pour la substance organique d'un alpha-hydroxyaldéhyde, d'un acide alpha-hydroxycarboxylique, d'un hydrate de carbone ou d'un glycoside.

2. Procédé selon la revendication 1, dans lequel la solution formée à l'étape b) est concentrée dans une étape c) par séparation d'eau (14) par osmose inverse et/ou évaporation partielle ou complète de l'eau (14).

3. Procédé selon une des revendications précédentes, dans lequel de l'eau supplémentaire et/ou l'eau (14) séparée à l'étape c) est ajoutée au mélange réactionnel (10) pour compenser l'eau séparée à l'étape a).

4. Procédé selon une des revendications précédentes, dans lequel une substance organique supplémentaire (12) est ajoutée au mélange réactionnel (10) pour compenser la substance organique transformée.

5. Procédé selon une des revendications précédentes, dans lequel l'état de contact et/ou la séparation en tant que vapeur (18) selon l'étape a) s'effectue à une température de 15 à 119,5°C, notamment 40 à 95°C, notamment 50 à 85°C, notamment 55 à 85°C, notamment 60 à 83°C.

6. Procédé selon une des revendications précédentes, dans lequel la séparation en tant que vapeur (18) selon l'étape a) s'effectue à une pression réduite par rapport à la pression atmosphérique.

7. Procédé selon une des revendications précédentes, dans lequel la condensation de la vapeur (18) selon l'étape a) s'effectue par introduction de la vapeur (18) dans de l'eau, une solution aqueuse ou la solution aqueuse selon l'étape b).

8. Procédé selon une des revendications précédentes, dans lequel l'hydroxyde (24) est l'hydroxyde d'ammonium, l'hydroxyde d'aluminium, l'hydroxyde de cuivre, l'hydroxyde de nickel, un hydroxyde de métal alcalino-terreux, un hydroxyde de métal alcalin, l'hydroxyde de calcium, l'hydroxyde de magnésium, l'hydroxyde de sodium, l'hydroxyde de potassium ou l'hydroxyde de césium.

9. Procédé selon une des revendications précédentes, dans lequel l'hydroxyde (24) à l'étape b) est utilisé dans un rapport stoechiométrique à l'acide formique tel qu'il est entièrement ou presque entièrement transformé.

10. Procédé selon une des revendications précédentes, dans lequel n = 3 + y.
